**Europäisches Patentamt**

**European Patent Office**

**Office européen des brevets**

(11) Numéro de publication : **0 457 656 A1**

(19)

(12)                           **DEMANDE DE BREVET EUROPEEN**

(21) Numéro de dépôt : **91401206.7**

(51) Int. Cl.⁵ : **C11D 3/48, A01N 47/44**

(22) Date de dépôt : **07.05.91**

(30) Priorité : **15.05.90 FR 9006039**

(43) Date de publication de la demande :
**21.11.91 Bulletin 91/47**

(84) Etats contractants désignés :
**BE CH DE FR LI LU NL**

(71) Demandeur : **EPARCO**
**78, rue de Provence**
**F-75009 Paris (FR)**

(72) Inventeur : **Gauthier-Fournier, François**
**Rue des Frères Argand, Résidence Baie**
**d'Azur**
**F-34140 Meze (FR)**

(74) Mandataire : **Derambure, Christian**
**BUGNION ASSOCIES 55, rue Boissonade**
**F-75014 Paris (FR)**

(54) **Composition de nettoyage et de désinfection.**

(57)     Composition de nettoyage et de désinfection
à usage ménager, caractérisée en ce qu'elle
comprend, en combinaison, de l'alcool gras
éthoxylé ; un co-tensioactif ; de l'alcool isopropylique ; du chlorhydrate de polyhexaméthylène biguanide ; du chlorure de didécyl
diméthyl ammonium ; du chlorure de benzalkonium.

EP 0 457 656 A1

Jouve, 18, rue Saint-Denis, 75001 PARIS

L'invention concerne des compositions de nettoyage et de désinfection à usage ménager, à propriétés hypoallergéniques et à capacités acaricides.

Les compositions de nettoyage et de désinfection des surfaces ou des liquides tels que murs, parois, éviers, tables, paillasses, etc... ont des caractéristiques de composition généralement déterminées par leurs conditions de mises en oeuvre. On connaît plus particulièrement les compositions de nettoyage et désinfection à usage ménager individuel ou collectif et celles à usage industriel. Les compositions à usage ménager doivent répondre notamment à des exigences de prix de revient et de sécurité. Des compositions à usage ménager connues du commerce sont le plus souvent à base d'eau de Javel. Le cas échéant la composition est présentée à la vente à l'état concentré et utilisée à l'état dilué à l'eau. Cependant l'efficacité désinfectante de telles compositions s'avère être particulièrement faible, surtout à l'état dilué. De plus elles présentent d'autres inconvénients (sécurité, odeur...). Les compositions à usage industriel requièrent généralement une efficacité supérieure avec, comme contrepartie un prix éventuellement supérieur. Par exemple, une telle composition connue à usage industriel comporte un tensioactif non-ionique, du butyl-glycol à titre de co-tensioactif et du chlorhydrate de poly(hexaméthylène biguanide) - tel que celui connu sous la marque VANTOCIL IB de la Société I.C.I à titre de biocide. Cependant d'une part l'efficacité désinfectante de telles compositions est également limitée et, d'autre part, ces compositions ne peuvent être employées à usage ménager.

On peut se référer, par ailleurs, aux documents suivants : EP-243 713 qui concerne une composition bactéricide comprenant, comme biocide, du chlorure de didécyl diméthyl ammonium ; UK-2 132 087 qui concerne une composition comprenant du chlorure de benzalkonium et des composés d'ammonium quaternaires ; JP-82 109899 qui concerne une composition comprenant un tensioactif non-ionique ; JP-82 09717 ; US-4 272 395 ; UK-1 562 961 ; UK-1 551 224 ; DE-2 810 998 ; DE-2 226 823 ; DE-1 923 889 ; FR-69 04494 ; FR-71 11177 ; FR-74 00681 ; FR-74 16119 ; FR-78 31058 ; FR-78 27929 ; US-45 40505 ; UK-795 814 ; DE-2 811 756 ; FR- 1 576 016 ; DE-3 639 635 ; WO-86 05 509 ; EP-99 209 ; WO-86 02 090.

Par ailleurs, la réglementation française sur les compositions de nettoyage et de désinfection à usage ménager ne permet l'usage que de certains produits ou composés en tant qu'éléments constituant la composition.

La norme AFNOR NFT 72171 permet de simuler les conditions de nettoyage et de désinfection simultanées lorsque la présence de matières organiques gêne l'activité désinfectante. Dans le cas de la mise en oeuvre de la norme avec 1 % d'albumine et 1 % d'extrait de levure en tant que "substance interférente" en présence de bactéries qualitativement et quantitativement connues et d'eau de javel à concentration voulue, l'abattement du nombre de germes, mesuré après cinq minutes, n'atteint pas le seuil fixé par la norme pour bénéficier de la qualification de "produit désinfectant". L'invention vise des compositions pouvant, au contraire de l'eau de javel bénéficier de cette qualification AFNOR "désinfectant bactéricide".

L'invention a donc pour objectif de proposer des compositions de nettoyage et de désinfection à usage ménager ayant une efficacité, notamment biocide, très substantiellement supérieure à celle des compositions à usage ménager connues à ce jour et, simultanément, répondant aux exigences réglementaires, commerciales, d'usage pratique auxquelles doivent répondre ces compositions. L'invention a aussi pour objectif que de telles compositions puissent être présentées à la vente à l'état concentré et utilisées à l'état dilué, sans que cette dilution n'affecte notablement leurs propriétés, notamment biocides, des compositions, au contraire. L'invention a enfin pour objectifs, que la composition bénéficie de la qualité de "désinfectant" selon la norme AFNOR NFT 72171 déjà citée, présente des propriétés hypoallergéniques et soit dotée de capacités acaricides.

A cet effet, l'invention propose, d'abord, une composition de nettoyage et de désinfection à usage ménager, caractérisée en ce qu'elle comprend, en combinaison, de l'alcool gras éthoxylé ; un co-tensioactif ; de l'alcool isopropylique ; du chlorhydrate de polyhexaméthylène biguanide ; du chlorure de didécyl diméthyl ammonium ; du chlorure de benzalkonium.

L'invention concerne donc une composition de nettoyage et de désinfection à usage ménager individuel ou collectif. A ce titre, cette composition répond aux exigences imposées par un tel usage : prix de revient convenable, sécurité assurée, conformité avec les règlements en vigueur.

La composition qui est maintenant décrite est principalement destinée à être présentée à l'état concentré, avec les avantages qui découlent d'une telle présentation, cette composition concentrée étant diluée à l'eau pour son usage effectif.

Une telle composition de nettoyage et de désinfection est destinée au nettoyage et à la désinfection des surfaces ou de liquides, dans la maison, pour différentes applications ou supports : murs, parois, éviers, tables, paillasses, etc..., cuisines, salles de bains, W.C ou autres...

La composition selon l'invention comprend en combinaison au moins un tensioactif non ionique, au moins un co-tensioactif choisi dans le groupe comprenant l'éther méthylique du dipropylène glycol et l'alcool isopropylique et, enfin, au moins un biocide cationique comprenant le chlorure de benzalkonium ou/et équivalent. De plus, cette composition comprend en outre de l'eau formant solvant, y compris sous sa présentation concentrée et, enfin le cas échéant, au moins un colorant et/ou un parfum.

Le ou au moins un des ou les tensioactifs non ionique utilisé dans la composition est un alcool gras à chaîne alkyl ramifiée ou non à au moins une et jusqu'à onze moles d'oxyde d'éthylène. Ainsi, la composition considérée comporte comme tensioactifs non ionique un alcool gras 70E combiné avec un alcool gras 30E, les essais réalisés ayant démontré qu'une telle combinaison donnait des résultats particulièrement efficaces et surprenants. Le cas échéant, un alcool éthoxylé (tensioactif non ionique) comporte additionnellement entre 1 et 10 oxydes de propylène (bornes comprises).

Un co-tensioactif est choisi dans le groupe comprenant l'éther méthylique du dipropylène glycol et l'alcool isopropylique. De plus, ce groupe exclut formellement le butyl-glycol.

Un biocide cationique est choisi dans le groupe comprenant outre le chlorure de benzalkonium ou/et équivalent, le chlorhydrate de polyhexaméthylène biguanide et le chlorure de didécyl diméthyl ammonium. En particulier, on utilise comme biocide cationique, la combinaison du chlorure de benzalkonium ou/et équivalent, d'un chlorhydrate de polyhexaméthylène biguanide et de chlorure de didécyl diméthyl ammonium.

Par exemple, un équivalent du chlorure de benzalkonium est choisi dans le groupe comprenant les chlorures ou bromures d'ammoniums quaternaires ayant une chaîne hydrocarbonée saturée, droite ou ramifiée, ayant de 8 à 18 carbone (bornes comprises) et dont le radical aryle correspond au groupement phényle ou au groupement benzyle. On peut citer les chlorures ou bromures de :

- alkyl triméthyl ammonium
- dialkyl diméthyl ammonium
- trialkyl méthyl ammonium
- alkyl aryl diméthyl ammonium
- alkyl éthylaryl diméthyl ammonium
- dialkyl aryl méthyl ammonium
- trialkyl aryl ammonium
- alkyl diaryl méthyl ammonium
- dialkyl diaryl ammonium
- alkyl diméthyl aryl phénoxy (ou crésoxy) éthoxy éthyl ammonium
- alkyl benzyl imidazolinium

ainsi que le chlorure (mais non le bromure) d'alkyl pyridinium.

On donnera maintenant les proportions ou les rapports de proportions de différents composants de la composition. Il est entendu que ces proportions ou rapports s'entendent en poids, cette précision n'étant plus donnée par la suite.

La composition telle qu'elle vient d'être décrite, à l'état concentré, peut comprendre jusqu'à 96 % d'eau, notamment de l'ordre de 42,4 %.

Elle peut comprendre entre 1 % et 50 % de tensioactifs (un ou plusieurs tensioactifs) notamment de l'ordre de 22,5 % pour les tensioactifs pris dans leur ensemble.

Elle peut comprendre également de 1 % à 25 % de co-tensioactifs, notamment de l'ordre de 14 %.

Enfin, elle peut comprendre entre 1 % et 40 % de biocide cationique, notamment de l'ordre de 20,1 %.

Le ratio d'alcool gras 70E par rapport à l'alcool gras 30E est compris entre 1,5 et 40 notamment et de l'ordre de 10,25. Le ratio d'alcool isopropylique par rapport à l'éther méthylique du dipropylène glycol est compris entre 0,33 et 3 notamment et de l'ordre de 1. Enfin, les ratios de chlorure de didécyl diméthyl ammonium et de chlorure de benzalkonium ou/et équivalent par rapport au chlorhydrate de polyhexaméthylène biguanide sont compris entre 0,1 et 20, notamment sont de l'ordre de respectivement 1,18 et 1,76.

D'excellents résultats ont été obtenus avec une composition de nettoyage et de désinfection qui comprend de l'ordre de 20,5 % d'alcool gras 70E ; de l'ordre de 2 % d'alcool gras 30E ; de l'ordre de 7 % d'éther méthylique du dipropylène glycol ; de l'ordre de 7 % d'alcool isopropylique ; de l'ordre de 5,1 % de chlorhydrate de polyhexaméthylène biguanide ; de l'ordre de 6 % de chlorure de didécyl diméthyl ammonium ; de l'ordre de 9 % de chlorure de benzalkonium ou/et équivalent.

Par exemple et conformément à un mode de réalisation possible de l'invention, on peut utiliser comme alcool gras les alcools gras polyéthoxylés commercialisés par la Société I.C.I FRANCE sous les marques SYMPERONIC A 7 et SYMPERONIC A 3.

Le co-tensioactif constitué par l'éther méthylique du dipropylène glycol peut être par exemple du DOWANOL DPM de chez DOW CHEMICAL.

Le biocide constitué par le chlorhydrate de polyhexaméthylène biguanide peut être à la concentration de 20 % et être constitué de VANTOCIL IB de la Société I.C.I France. Quant au chlorure de didécyl diméthyl ammonium, il peut être à 50 % et constitué par du BARDAC 22 de la Société LONZA FRANCE. En ce qui concerne le chlorure de benzalkonium, il peut être à 50 % et constitué par VITALUB QC 50 de la Société VALLUY.

Il va de soi que l'on pourrait envisager des variantes de compositions qui entreraient dans le cadre de la présente invention. Tel serait le cas de compositions dont les composants seraient strictement équivalents à ceux décrits ou très voisins. En particulier et en ce qui concerne les tensioactifs, on pourrait utiliser des alcools gras ayant un nombre d'éthoxylation différent de ceux - 3 et 7 - mentionnés. Par exemple, ce nombre d'éthoxylation pourrait être compris entre 1,5 et 11.

Une composition telle que celle qui vient d'être décrite peut donc se présenter sous forme concentrée avec les avantages qui découlent d'une telle présentation et être diluée à l'eau pour l'usage. Par exemple, la dilution peut aller jusqu'à 8g de la composition par litre le degré de dilution dépend notamment de l'usage envisagé pour la composition.

## Revendications

1. Composition de nettoyage et de désinfection à usage ménager, caractérisée en ce qu'elle comprend, en combinaison, de l'alccol gras éthoxylé ; un co-tensioactif ; de l'alcool isopropylique ; du chlorhydrate de polyhexaméthylène biguanide ; du chlorure de didécyl diméthyl ammonium ; du chlorure de benzalkonium.

2. Composition selon la revendication 1, caractérisée en ce qu'elle comprend, en outre, de l'eau formant solvant.

3. Composition selon l'une quelconque des revendications 1, 2, caractérisée en ce qu'elle comprend, en outre, au moins un colorant et/ou parfum.

4. Composition selon la revendication 1, caractérisée en ce qu'un alcool gras éthoxylé comporte additionnellement au moins un oxyde de propylène.

5. Composition selon la revendication 1, caractérisée en ce que le chlorure de benzalkonium est au moins partiellement remplacé par un corps choisi dans le groupe comprenant les chlorures, les bromures d'ammonium quaternaires ayant une chaîne hydrocarbonée saturée, droite ou ramifiée, ayant de 8 à 18 carbone, et dont le radical aryle correspond au groupement phényle ou au groupement benzyle, à l'exception du bromure d'alkyl pyridinium.

6. Composition selon la revendication 2, caractérisée en ce qu'elle comprend de l'ordre de 42,4 % d'eau, en poids.

7. Composition selon la revendication 1, caractérisée en ce que l'alcool gras éthoxylé est une combinaison d'alcool gras 70E et d'alcool gras 30E.

8. Composition selon la revendication 1, caractérisée en ce que le cotensioactif est l'éther méthylique du dipropylène glycol et exclut le butyl-glycol.

9. Composition selon l'une quelconque des revendications 1 à 8 caractérisée en ce qu'elle comprend entre 1 % et 50 % d'alcool gras, en poids, notamment de l'ordre de 22,5 %.

10. Composition selon l'une quelconque des revendications 1 à 9, caractérisée en ce qu'elle comprend entre 1 % et 25 % en poids d'alcool isopropylique.

11. Composition selon l'une quelconque des revendications 1 à 10, caractérisée en ce qu'elle comprend entre 1 % et 40 % en poids de chlorhydrate de polyhexaméthylène biguanide, de chlorure de didécyl diméthyl ammonium et de chlorure de benzalkonium.

12. Composition selon la revendication 7, caractérisée en ce que le ratio, en poids, d'alcool gras 70E par rapport à l'alcool gras 30E est compris entre 1,5 et 40 notamment de l'ordre de 10,25.

13. Composition selon les revendications 1 et 8, caractérisée en ce que le ratio, en poids, d'alcool isopropylique par rapport à l'éther méthylique du dipropylène glycol est compris entre 0,33 et 3, notamment de l'ordre de 1.

14. Composition selon l'une quelconque des revendications 1 et 13 caractérisée en ce que les ratios, en poids, de chlorure de didécyl diméthyl ammonium et de chlorure de benzalkonium par rapport au chlorhydrate de polyhexaméthylène biguanide sont compris entre 0,1 et 20, notamment de l'ordre de respectivement 1,18 et 1,76.

15. Composition selon l'une quelconque des revendications 1 à 14, caractérisée en ce qu'elle comprend en poids de l'ordre de 20,5 % d'alcool gras 70E ; de l'ordre de 2 % d'alcool gras 30E ; de l'ordre de 7 % d'éther méthylique du dipropylène glycol ; de l'ordre de 7 % d'alcool isopropylique ; de l'ordre de 5,1 % de chlorhydrate de polyhexaméthylène biguanide ; de l'ordre de 6 % de chlorure de didécyl diméthyl ammonium ; de l'ordre de 9 % de chlorure de benzalkonium ou/et équivalent.

16. Composition selon l'une quelconque des revendications 1 à 16, caractérisée en ce qu'elle se présente sous forme concentrée destinée à être diluée à l'eau pour l'usage.

17. Composition selon la revendication 17, caractérisée en ce qu'elle est diluée à l'eau jusqu'à 8g par litre environ de manière à pouvoir être utilisée.

Office européen
des brevets

## RAPPORT DE RECHERCHE EUROPEENNE

Numero de la demande

EP 91 40 1206

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 099 209 (SURGIKOS INC.) <br> * En entier * <br><br> --- | 1-6,10, 11,16, 17 | C 11 D 3/48 <br> A 01 N 47/44 |
| A | EP-A-0 041 448 (N. SALKIN) <br> * En entier * <br><br> --- | 1 | |
| A | EP-A-0 226 081 (HENKEL) <br> * Exemples; revendications * <br><br> ----- | 1 | |

DOMAINES TECHNIQUES
RECHERCHES (Int. Cl.5)

C 11 D
A 01 N

Le présent rapport a été établi pour toutes les revendications

| Lieu de la recherche | Date d'achèvement de la recherche | Examinateur |
|---|---|---|
| LA HAYE | 13-08-1991 | GOLLER P. |

EPO FORM 1503 03.82 (P0402)